# EUROPEAN PATENT SPECIFICATION

(11) **EP 2 377 060 B1**
(45) Date of publication and mention of the grant of the patent: **03.04.2019**
(21) Application number: 09807602.9
(22) Date of filing: 15.12.2009
(51) Int. Cl.: G16H 80/00

(54) **SYSTEM AND METHOD FOR MULTI-PERSON AND MULTI-SITE, INTERACTIVE TREATMENT SIMULATION**
SYSTEM UND VERFAHREN ZUR INTERAKTIVEN BEHANDLUNGSSIMULATION MIT MEHREREN PERSONEN UND MEHREREN STANDORTEN
SYSTÈME ET PROCÉDÉ DE SIMULATION INTERACTIVE MULTI-INTERVENANTS ET MULTI-SITES D'UN TRAITEMENT

(30) Priority: 15.12.2008 GB 0822809
(43) Date of publication of application: 19.10.2011
(73) Proprietor: Dentsply Implants NV, 3500 Hasselt (BE)
(72) Inventor: VAN LIERDE, Carl, B-9402 Meerbeke (BE); MEYHI, Jo, B-3012 Wilsele (BE)
(74) Representative: IPLodge bvba
(86) International application number: PCT/EP2009/067229
(87) International publication number: WO 2010/079056

(56) References cited:
- US-A1- 2005 148 830
- US-B1- 6 587 828

## Description

### FIELD OF THE INVENTION

The present invention relates to systems, methods, devices and software for multi-person and multi-site, interactive treatment simulation, especially for dental treatment.

### BACKGROUND TO THE INVENTION

Dental treatment planning often uses one or more digitizing modalities such as Computerized Tomography (CT), Magnetic Resonance Imaging (MRI), optical scanning (e.g. laser light or white light) or 2D X-ray to visualize and evaluate the oral tissues of a patient. Over the last few years several (software) tools (e.g. SimPlant, Materialise Dental) have been commercialized to provide clinicians with means to define the best clinical approach on the basis of the obtained digitized information i.e. to plan or simulate treatment. These tools allow representations of artificial elements (e.g. implants, grafts) to be graphically superimposed on the images. Using image-processing techniques such as image segmentation, 2D or 3D representations of the anatomical structures (e.g. bone, teeth, blood vessels) can be derived from the data. In the software planning environment, the practitioner simulates treatment, determining desired positions and orientations for artificial elements, identifying optimal cutting paths, evaluating functional and aesthetical repercussions of tissue alterations, etc. Afterwards, the treatment simulation can be transferred to the patient using custom made personalized devices such as drill guides, surgical splints, aligners and the like.

In the past, cases for which this type of simulation is recommended would almost exclusively have been conducted by dental specialists with competences in one or more dental disciplines such as orthodontics, oral and maxillo-facial surgery, prosthodontics, etc. General practitioners (GP) typically referred patients to these specialists to undergo specialist procedures. To improve information exchange and case management between the parties involved (e.g. GP, specialist, dental lab) several systems and methods were suggested. US patent 5579393 describes a method for secure dental record interchange and US 7363239 describes an interface configured to receive and transmit ordering and manufacturing data from a plurality of users of different dental treatment systems. According to the latter patent, at least one coordinating adapter unit receives and transmits the ordering and manufacturing data and identifies from which dental treatment system the data comes. A communications network permits communication among elements of the system. Patent application WO 2009/029672 describes communication of patient information between dental practitioners, such as GP and specialist, over a network, and granting access to these data to selected users.

Other patents (e.g. US6345260, US2004102983, US2005027580) discuss remote appointment scheduling systems utilizing the internet.

On the commercial side, marketed software tools such as Cercon Coach (DeguDent, Germany), Collaborator and DDS weblink (DDS Ventures Inc, USA) provided dentists with tools for improved laboratory communication, electronic case referral and real time messaging to enable secure and documented communications of online patient requisite information. Patient data, files and digital images were immediately accessible through a web site and progress of patient care was automatically tracked.

Despite the availability of good tools to stimulate and facilitate collaboration between GP's and specialists, there has been a marked increase in competition between general practitioners (GP) and specialists in recent years. Since specialist services often are more profitable compared to GP work they hold an appeal over dentists wishing to maximize earnings for their practice. Fueled by aggressive marketing campaigns recruiting GP's to perform specialist procedures the dental market has seen a drastic increase in general dentists deciding to install implants, treat malocclusion and even place bone grafts. While computer assisted treatment simulation has been promoted and is being used by GP's to more confidently venture onto specialist terrain, there is a growing concern, both among industry experts and governmental agencies about the quality of care provided by dental practitioners who may be practicing specialist dentistry beyond the limits of their competence. In March 2006 the UK's *General Dental Council* (GDC) convened a working group to consider training standards for general dental practitioners who wish to practice implant dentistry. In the United States, the *Institute of Dental Implant Awareness* highlighted that referring dentists must be educated with regards to appropriate minimum requirements for surgical training should they wish to place their own implants. Also related to implant dentistry, Dr. Jay Malmquist, president of the *American Association of Oral and Maxillofacial Surgeons* has stated that the number of complaints filed by the patients injured or otherwise harmed by dental professionals lacking the necessary competences to perform a particular procedure is increasing. Among the expertise often lacked by GP's, industry experts cite a lack of understanding of
(i) clinical assessment of a patient's suitability for specialist treatment;
(ii) main treatment options available and their indications and contraindications for certain patient groups;
(iii) clinical and laboratory techniques used to obtain oral rehabilitation.

This problem has persisted for a number of years now. While the use of treatment simulation software and the use of custom made transfer devices can be taught by means of a two or three day course, the clinical expertise required for specialist treatments takes several months or even years to acquire and master. Promulgated standards for training, providing proper education to GPs about risk management and potential complications associated with specialist treatment may offer a solution, but as of yet are not available.

Another method to overcome the problem is to have the software generate treatment plans. US patent 7003472 describes a solution directed towards the orthodontic field where specialist know-how is incorporated in a computer apparatus for generating such treatment plans. The method and apparatus are characterized in that the patient treatment plan is generated by processing digital input information provided by the user. The *art* of treatment is thereby transformed in *calculations* practiced as a series of software instructions which are executable on a workstation. The workstation can be installed at the site of treatment of the patient, such as in an orthodontic or dental clinic. This approach assumes that treatment planning can be reduced to an almost binary decision making process and thereby disregards benefits attributable only to the experience of a seasoned specialist. Moreover, the output (i.e. treatment plans) generated by such a method and computer apparatus always needs to be verified by a clinically trained professional, making it less useful for real novices to the specialization, which lack the competence to interpret the treatment plan relative to its adequacy.

Similar ideas have been mentioned in US patent applications 2001002310 and 2004137400 where a computer is used to create a plan for repositioning an orthodontic patient's teeth. The computer receives an initial digital data set representing the patient's teeth at their initial positions and a final digital data set representing the teeth at their final positions. The computer then uses the data sets to generate treatment paths along which teeth will move from the initial positions to the final positions. Alternatively, the treatment plan is selected from a tooth treatment pattern from a library of predetermined tooth treatment patterns; and generated by implementing the selected tooth treatment pattern. Again, the limitation of these systems lies in the elimination of a human actor in the treatment planning process.

The limitations and disadvantages related specifically to traditional case referral on the one hand and GP's performing specialist dentistry on the other, are clear. What is more though, is that neither approach is organized around optimal comfort, success and convenience for the patient. In traditional case referrals patients may be required to travel, leaving the comfort of the familiar local dentist office to receive proper treatment. Even so, a patient is very unlikely to benefit from expertise available half way across the globe. Being treated by a single GP obviously is more practical for the patient but then again raises questions about the adequacy and quality of the treatment.

### SUMMARY OF THE INVENTION

The present invention provides alternative systems, methods, devices and software for multi-person and multi-site, interactive treatment simulation, especially for dental treatment.

Embodiments of the present invention have the advantage of overcoming at least one the above-listed problems of the background art. An advantage of the present invention is that in some embodiments at least some of the disadvantages and limitations of earlier suggested solutions can be avoided. The present invention provides a system and method for multi-person and multi-site, interactive medical especially dental treatment simulation. The system is preferably implemented over a communications network such that the system allows a group of planners to provide advice on a patient's suitability for specialist treatment; to interactively simulate treatment in function of known indications or contraindications and to recommend the best laboratory techniques to obtain optimal oral rehabilitation. The computer simulation is recorded by the system and updated with the current status information as the treatment simulation is modified. Service providers need however not be working concurrently.

The system is especially suitable for dental treatment, e.g. by a dentist, or a dental surgeon optionally advised by one or more dental specialists.

The system comprises computer based processors and memory, both volatile and non-volatile as required to carry out the operations of the system as detailed below.

A primary advantage of this system is that any number of specialists, such as dental specialists, can be involved in to instruct the GP, e.g. a dentist, via treatment simulations while the GP can focus on administering the treatment. The level of training required for the GP is less and specialist competences can be acquired gradually without putting patients at risk.

An added advantage of the invention is that even specialists can choose to focus on either the simulation of treatments or on the clinical act of performing the intervention (i.e. administrating the treatment). Specialists having a strong affinity with computers and software applications can thereby perform tasks which are burdensome and/or difficult for colleagues and visa versa. US6587828 discloses a method and apparatus for generating a patient treatment plan which includes processing that begins by providing a list of health care services to a patient and/or care provider. The processing continues by prompting for input of digital information regarding the patient when a health care service has been selected. The processing continues by determining whether a sufficient amount of digital information has been received. If so, the processing continues by simulating treatment of a patient based on the digital information, a treatment objective, and normalized patient data. The processing then continues by generating the patient treatment plan in accordance with the simulating of the treatment when the simulated treatment results have been acknowledged.

US2005/148830 computerized medical and dental diagnosis and treatment planning and management system. The system includes a menu based computer program with fields for entering information including answers to questions and measured data related to a plurality of different types of examinations, wherein the entered information in specific fields on specific forms for one type of examination will automatically populate selected data fields in a series of other forms for other types of examinations. The forms are electronically disseminated from a central system by one treating practitioner to other practitioners who render service to a patient.

### SUMMARY OF THE INVENTION

The present invention provides alternative systems, methods, devices and software for multi-person and multi-site, interactive treatment simulation, especially for dental treatment.

Embodiments of the present invention have the advantage of overcoming at least one the above-listed problems of the background art. An advantage of the present invention is that in some embodiments at least some of the disadvantages and limitations of earlier suggested solutions can be avoided.

The present invention provides a system and method for multi-person and multi-site, interactive dental treatment simulation according to claims 1 and 7 respectively. The system is implemented over a communications network such that the system allows a group of planners to provide advice on a patient's suitability for specialist treatment; to interactively simulate treatment in function of known indications or contraindications and to recommend the best laboratory techniques to obtain optimal oral rehabilitation. The computer simulation is recorded by the system and updated with the current status information as the treatment simulation is modified. Service providers need however not be working concurrently.

The system is especially suitable for dental treatment, e.g. by a dentist, or a dental surgeon optionally advised by one or more dental specialists.

The system comprises computer based processors and memory, both volatile and non-volatile as required to carry out the operations of the system as detailed below.

A primary advantage of this system is that any number of specialists, such as dental specialists, can be involved in to instruct the GP, e.g. a dentist, via treatment simulations while the GP can focus on administering the treatment. The level of training required for the GP is less and specialist competences can be acquired gradually without putting patients at risk.

An added advantage of the invention is that even specialists can choose to focus on either the simulation of treatments or on the clinical act of performing the intervention (i.e. administrating the treatment). Specialists having a strong affinity with computers and software applications can thereby perform tasks which are burdensome and/or difficult for colleagues and visa versa.

Based on the computer simulation of a treatment the GP can order personalized devices enabling him to administer the treatment on the patient.

. The present invention also includes a computer program according to claim 14 comprising instructions for carrying out the method of claims 7 to 13. Further, the present invention includes a machine readable signal recording device according to claim 15 on which is stored the computer program. Examples of computer readable signal bearing media include: recordable type media such as magnetic disks, e.g. floppy disks or hard disks; or optical disks such as CD ROMs, DD-ROMs; or solid state memory such as Random Access Memory, USB memory sticks, flash memory; or magnetic tape storage media; or transmission type media such as digital and analogue communication links.

### BRIEF DESCRIPTION OF THE DRAWINGS

Embodiments of the invention will be described, by way of example only, with reference to the accompanying drawings in which:
Fig. 1 shows an embodiment of a system in accordance with the invention.

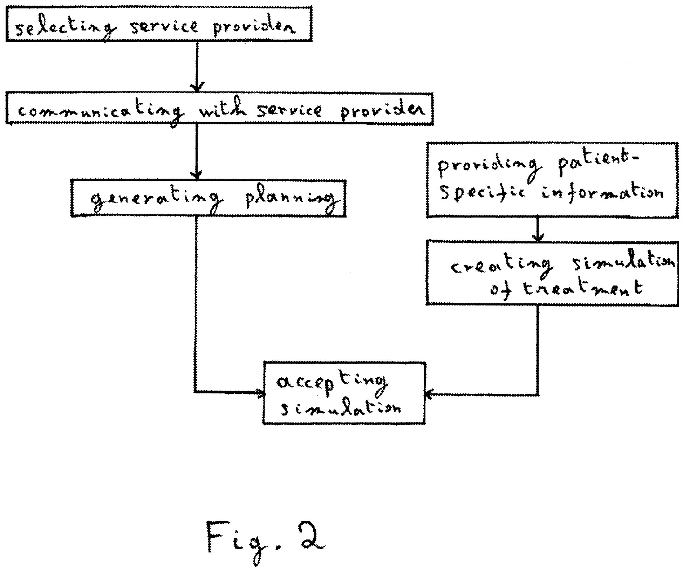

Fig. 2 shows a flowchart of a particular embodiment of a method in accordance with the invention. In this particular embodiment, the steps of the method are given in a specific order. The invention is not restricted to this specific order; the scope of the invention is defined by the appended claims.

### DESCRIPTION OF PREFERRED EMBODIMENTS

In the following detailed description of the preferred embodiments, reference is made to the accompanying drawings, which form a part hereof, and within which are shown by way of illustration specific embodiments by which the invention may be practiced. The drawings described are only schematic and are non-limiting. In the drawings, the size of some of the elements may be exaggerated and not drawn on scale for illustrative purposes. Those skilled in the art will recognize that other embodiments may be utilized and structural changes may be made without departing from the scope of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

According to a preferential embodiment the invention discloses a system, preferably implemented over a data communications network, and a method for creating an individualized computer simulation of a treatment using a multi-site / multi person interactive approach.

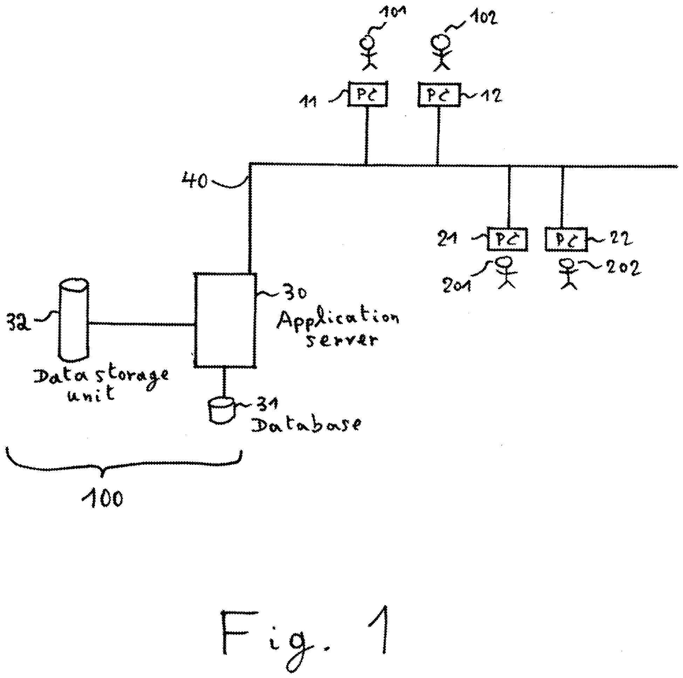

Referring now to Fig. 1, the inventive system (100) includes a data storage unit (32) for storing digitized information of the patient anatomy (e.g. CT images), the created simulations and any other relevant information (e.g. library of artificial elements, patient history, etc). This storage unit (32) can for instance be a file storage server. The system (100) also includes a database (31) of registered users (GP's, specialists, scan centers) including -when applicable-details about their geographical location, contact information, credentials, service fees, etc. According to a preferred embodiment the database is kept on an application server (30) having a processor and memory that communicates with the file storage server (32) (having a processor and memory) via web-services. The application server (30) can further communicate with other servers (such as an online shop), with production management systems and with hardware via such web-services. Communication with scanning equipment may also be conducted via PACS. The system (100) can further be configured to exchange information with computer systems used for accounting or used by insurance providers and social security providers.

The system uses computer based human readable pages on a public network, e.g. a wide-area data network (40) such as the World Wide Web where information can be accessed, downloaded or viewed. Typically, a browser (e.g. Internet explorer supplied by Microsoft Corp. USA) is used for communication between the application server (30) and remote personal computers (11,12,21,22) (from the GP's (101,102) or experts (201,202)). Access to the human readable pages may be limited to people having appropriate access rights (e.g. ID-code, username and password).

The system provides GP's (General Practitioners) with the means to select one or more preferred service providers. This can for example be done by allowing the GP to indicate providers from a drop down menu. Such a drop down menu is generated by the system via queries run on its database(s), based on search criteria entered by the GP on the webpage. Alternatively, the system stores default providers as entered by the GP or based on a "most frequently used" criterion.

The system preferentially checks the availability (e.g. workload, calendar) of the planners and notifies the GP of any conflicts relative to a specified deadline. The system also preferentially allows capacity reservation to be made. As an example, the application server may communicate with service providers' software calendars via exchange servers or systems such as Lotus group wear. To link with practice management software web-services can be developed or import-export tools (typically via .xml files) must be created.

The system shows the information stored in the database about the service providers on a webpage (human readable page). Having been selected by the GP, the designated service providers are informed by the system, for instance via e-mail, that a job has been assigned to them. The service provider can, via a simple reply, decline or accept the job. If no reply is given within a predetermined timeframe, the GP is informed automatically (e.g. by e-mail, short message service (SMS) etc.) and can select a different service provider for the job. If the provider accepts the job, he receives a system-generated ID code granting him/her access to the necessary case information.

According to a preferential embodiment, the simulation environment (software) is integrated in the system. The digital information of the patient is thereby displayed in a webpage and treatment simulation can be performed from within the web-application. The system provides the simulation functionally via the public network. Operations on or in function of the digital information (e.g. image processing, artificial elements, simulating cutting paths, etc.) are remotely conducted on a central machine e.g. the application server.

Alternatively, the simulation environment is not included as part of the system. It can for instance be separate software running on a computer that merely is linked up to the system. In such a case, the digital information must be transferred from the system to the separate simulation software. The system updates the status of the treatment simulation by synchronizing the treatment simulation stored on its storage unit, with the one being created in the separate environment. The system monitors the differences between the two simulations and does not require the entire digital information to be exchanged, therefore drastically reducing the amount of data exchange required.

The system can store treatment simulation of different service providers separately (i.e. versioning). The system can also allow multiple experts to work on a single simulation as a team. In such cases, it may store incremental versions until the final simulation has been obtained. Experts can thus easily revert back to previous versions of the simulation.

After the simulation has been created the system notifies the GP, who can than review it for acceptance. The information contained in the simulation may also be captured automatically in a document e.g. a .pdf file and sent to the GP via e-mail or by other means. The system keeps track of whether the simulation has been accepted or not. Upon acceptance, fees can be charged by the expert(s) to the GP. According to a preferred embodiment of the invention, the system generates a single invoice for the GP detailing the costs associated with the efforts of the individual service providers. The determination of costs is based on the pricing information stored in the database, which is not necessarily limited to a fixed fee. The costs may for example be calculated on a time and materials basis. The system in such cases can for instance register the amount of time spent on a simulation by the expert. The system tracks the payment of the invoice on a dedicated account and distributes the money over the service providers in function of their contributions. The system may be fit with measures to protect service providers in case payments are not made. For example, per case a fixed transaction fee can be charged to build-up a monetary reserve used to compensate duped service providers.

According to another feature of the invention, the system monitors through the use of processors the activity by the service provider i.e. planner-specialist relative to a deadline for planning specified by the GP. The system notifies both the service provider and GP when certain time points have been exceeded prior to the deadline without expected activity (e.g. viewing of the digital information) having taken place. In a preferred embodiment, the system records and stores in non-volatile memory information about the reliability of the service providers as well as other data relevant as measures to evaluate performance (e.g. number of plans created, GP satisfaction with the provided services, average time to produce the treatment plan, etc.). Similarly, the system may record and store in non-volatile memory information characterizing the GP as customer (e.g. number of treatment plans requested, trustworthiness with respect to payments, feedback from planners, etc.). The information about service providers and GP's may, according to one embodiment, automatically be displayed via a webpage.

According to the preferred embodiment, the system recognizes and groups the digital information (e.g. images) belonging to the same patient when said information is loaded onto its storage unit. The system may accomplish this for instance by reading and analyzing the header information in the images and sorting the images based on that information. Several digital information sets from different patient can thereby be loaded onto the storage unit simultaneously.

According to another embodiment of the invention, the system only displays or provides access to the digital information of the patient (i.e. images, patient history and the like) in an anonymous manner to people other than the person requesting services. The system thereby removes any information allowing identification of the patient (e.g. name, address, social security code, etc.) when said digital information is for instance viewed or downloaded from the storage unit. According to a preferred way of working, the system generates a unique identification code per case which it assigns (for instance by means of a digital tag) to the digital information when viewed, downloaded or otherwise accessed by parties other than by the GP. The system records the relation between the unique ID codes (tags) and the patient's personalia, allowing at all times to link the appropriate treatment plan to the correct patient. The system may have built-in fail-safes to assure integrity of the records.

According to yet another embodiment of the invention, the system allows outsourcing services other than treatment simulation such as acquiring the digital information about the anatomy of the patient (e.g. scanning of the patient) or lab services which may include designing and manufacturing temporary dental restorations, orthognatic and orthodontic appliances, crowns, bridges, etc.

Also according to the preferred embodiment of the invention the system registers, based on the computer simulation, which hardware tools, artificial elements and the like are required for the GP to perform the clinical intervention. The system may be linked to an online ordering system to automatically generate an order for said tools and artificial elements. For the GP this eliminates the need to keep a stock of components, instead ordering only what is needed for a single intervention. Alternatively the system could also be amended with a centralized stock management system, integrated with the database of GPs to keep track per GP the amount of different hardware components still available in the office and to automatically notify the GP when the stock reaches a critical threshold. The system may even recommend appropriate products for specific procedures, based on the simulation generated by the specialist.

### Illustrative examples

According to a first example, the system and method are configured and used to create a custom, personalized computer simulation for the placement of dental implants in the jaw of a patient. The simulation according to this example may consist of at least one or more of the following components: a digital file uniquely determining the positions of the implants relative to the anatomy (e.g. jawbone) of the patient; a list (digital or hard copy) of components required according to the simulation (e.g. implants including type, brand, length, diameter; abutments; other prosthetic components); a comprehensive overview (e.g. by means of a picture of the jaw with annotations) of which components to use per implant; a lab prescription detailing the prosthetic build-up to be created on top of the implants; etc.

In this example, a GP can navigate via e.g. a web browser to a website -which is part of the system - where he/she can establish an account by filling out an online registration form. The account is associated with an entry in the database of the system, identifying the GP as a customer (as opposed to a service provider) and storing the administrative information (e.g. name, address, billing address, telephone number, fax, etc.) filled out by the GP in the registration form. The account is protected e.g. by means of a user name and a password. Filling out the correct user name and password on the website, acts as a security clearance allowing the GP to work from the associated account and granting him/her access to functionality provided by the system. Via the system, the GP can then assemble a dedicated team of experts to perform specific tasks leading to the creation of a patient specific computer simulation of the dental implant treatment. Furthermore, the system coordinates (time-wise) the efforts of the experts (e.g. by tuning schedules or sending notifications). The system continuously monitors and updates the critical path, thereby informing the GP of the status of the case.

Different steps for generating a customized dental implant simulation may include, but are not limited to: creating a diagnostic tooth setup representative of the final dental restoration, making a radio-opaque scan template on the basis of said set-up; acquiring image information (e.g. CT) of the jaw(s) of the patient; making and digitizing gypsum casts or impressions of the existing dentition; formatting the image data as a file readable by an implant simulation software; creating a dental simulation via said software; designing a guide conform the simulation; designing a fixed or removable dental prosthesis incorporating attachments aligned with the implants according to the implant simulation.

The GP can choose to work without or with one or more service providers for each step. Service providers may be identical for more than one step. To indicate the desired services and to identify preferential experts, the GP must provide input to the system. This can be done by filling out an online form. Preferences per GP can be stored in the system. Via a webpage, the GP can (indirectly) access the system's database of service providers to make a choice. The system provides functionality (such as queries) to limit the subset of service providers per step based on one or more criteria (e.g. geographic location, price, credentials). The system may further assist the GP by proposing a team of experts to conduct the entire set of tasks based on criteria as shortest overall lead time; least amount of experts involved; cheapest solution; best qualified experts, etc.

When selecting experts, the system updates the total cost of the services being requested. Upon acceptance, the service providers are informed of the pending case and the necessary capacity / time reservations are communicated to them and/or directly entered in their schedules. The system may therefore be equipped with remote scheduling functionality.

The system generates a unique identification tag (e.g. code, password, number sequence, or the like) for the case. The tag identifies the account and the case, and is used to restrict access to the case information to those service providers that are part of the team selected by the GP. The tag may consist of more than one part e.g. a common part related to the case and a service-specific part to further restrict access among team members to information only available during particular steps.

Imaging centers engaged in the case via the system will thus be notified of appointments with patients, whom will subsequently undergo CT scans or the like. The medical image data thereby acquired will be fed back into the system e.g. by uploading the image files on a server. Typically this will be performed in two stages: firstly the files are uploaded to a temporary file storage unit on the application server, in a second stage they are transferred to a file storage server. Prior to this transfer, the system performs a check to verify and validate the completeness of the data. This can be done by a so called hash, which a code generated based on calculations that take the contents of the data into account. Only if the result of the calculations i.e. the code is identical before upload at the side of the service provider and after the upload on the storage unit, will the file be deemed complete.

Once the transfer is completed, an entry is made in the case file on the application server about the location of the digital information on the file storage server.

In some cases the imaging centers may have already formatted the data in a proper manner for it to be read by the implant simulation software. Only the resulting file will typically be transferred onto the system in such cases.
In more complex cases, dental labs may be involved as service providers to digitize plaster models of the dentition and register them onto the CT scan images to obtain a file combining the information acquired via different imaging modalities (e.g. volumetric imaging and surface scanning).

As soon as the image data has been converted and is in the appropriate file format and stored on the system, the status of the case is changed which acts as a trigger to notify the GP as well as the clinical expert(s) involved. The latter can start with the determination of the most optimal positions for the implants and abutments, in function of biomechanical (e.g. quality of the bone) and aesthetic (e.g. smile line of the patient) considerations. The system incorporates online implant simulation software and instant online communication tools (e.g. online meeting, chat-room, internet-telephony) and integrates the required hardware (e.g. webcams). As an example, web-links are created by the system that automatically contact the appropriate person via existing services such as Skype and like. Several people (for example an expert and the GP or a number of experts) can work on the simulation simultaneously while discussing the case. Alternatively they work on the case one after another until all agree on the final simulation of implant and abutment positions, orientations and the like. The system keeps track of the history (i.e. intermediate versions of the simulation) at least until the GP has accepted the final simulation.

Based on the approved simulation, the system inventories the components and tools (e.g. surgical kits, drills, personalized guides, etc.) required to treat the patient. The digital list can be printed out or can be used as an input for an online ordering service included in the system. The functionality provided by said service automatically groups components obtainable from the same supplier and prepares one or more separate orders that can be transferred online to the appropriate suppliers. The system therefore incorporates a database of suppliers with associated product offerings, prices, etc.

According to the example, the system is fitted with a remotely accessible digital archive, which can be used to store image data and/or other patient information (e.g. pictures). Alternatively archiving is done on external hard drives, DVDs or other data carriers. In such a case the system includes functionality e.g. on the application server to export case information for archiving purposes. In short, the appropriate digital information is retrieved from the file storage server, bundled with the case information on the application server and made available for writing onto appropriate archiving media.

According to a second example of the invention, the method and system are used to create a patient-specific orthodontic simulation. In particular, the system integrates different imaging equipment such as cone beam computed tomography scanners, intra-oral scanners, lateral X-ray equipment etc. The output of these devices, which can be located at different service providers, is directly uploaded to a secure section of the system's storage unit. Such a section is automatically created by the system as soon as the GP creates a new case via his account on the webpage. According to the example one or more different simulation software (2D and / or 3D) products may be used by at least one expert / service provider. For instance, one service provider may focus on simulation in lateral X-ray images, performing orthodontic analyses (e.g. Steiner, Ricketts), while another focuses on simulation in 3D e.g. determining the desired arch of the dentition from a functional and aesthetic point of view. By accessing his account, the GP can check the most recent status of his/her cases at any time. Similarly, service providers are provided with an overview of cases for which their services have been requested.

The GP can review the simulation(s) proposed by the experts. Before the simulation, the GP may have defined the type and brand of orthodontic appliances (e.g. brackets and wires) on the basis of which the simulation needs to be performed. The simulations typically will be presented to him for instance in the format of a picture of the predicted outcome or a movie simulating the movement of the teeth from their initial positions to their desired positions. As part of the simulation the system provides expert with software functionalities to create an un-ambiguous, standardized lab-prescription detailing the positions of retention braces, springs, etc. for any orthodontic appliances needed for the treatment. Said functionalities may be provided (i) in the form of a template document to be filled out online, (ii) as dedicated software for creating a 2D drawing of the appliance using (self-explaining) icons to represent certain features (e.g. hooks, coils) or (iii) as software for 3D design of the appliance.

Yet other examples make use of the system and method for creating custom treatment plans in the field of endodontics, prosthetics or even oral and maxillofacial surgery. In some cases, the system may also be used by experts to educate the GP about the clinical protocol to be followed when treating the patient (e.g. guidelines on anesthetic to be used, type of incision to be made, recommendations relative to the use of a drill guide etc.).

## Claims

1. A computer based system (100) for interactive simulation of a dental treatment to be administered on a patient, the computer system (100) being coupled to a communications network (40) and comprising a data storage unit (32) for storing patient-specific information, the computer system (100) comprising:
• means for selecting at least one dental specialist (201, 202) by a dentist (101, 102);
• means for communicating with said at least one dental specialist (201, 202) by the dentist (101, 102) over said communications network (40);
• means for generating a planning for the dental treatment;
• means for retrieving said patient-specific information from said data storage unit (32);
• means for creating a simulation of the dental treatment based on said patient-specific information;
• means for providing instructions to said dentist, said instructions being the dental treatment simulation provided by said at least one dental specialist;
• means for accepting the simulation by the dentist (101, 102), and
• means for generating, based on said accepted simulation, an inventory of devices required for said dental treatment **characterized in that**:
the system incorporates online implant simulation software and online communication tools; and
the simulation being performed by interactively simulating treatment in function of known indications or contraindications comprising a determination of the most optimal positions for implants and abutments, in function of biomedical and aesthetic considerations.

2. Computer based system according to claim 1 wherein said dentist and said at least one dental specialist are located at different sites.

3. Computer based system according to any previous claim further comprising:
• means for reviewing said simulation of said dental treatment;
• means for declining said simulation of said dental treatment;
• means for modifying said declined simulation of said dental treatment, or
• access control to said patient-specific information, or
• the inventory of devices being surgical kits and personalized guides, required for said dental treatment.

4. Computer based system according to any previous claim further comprising means for proposing at least one preferred dental specialist to said dentist.

5. Computer based system according to any previous claim further comprising access control to said patient-specific information.

6. Computer based system according to any previous claim wherein said communications network (40) is a wide area network.

7. A computer based method for interactive simulation of a dental treatment to be administered on a patient, the method comprising the steps of:
• selecting at least one dental specialist (201, 202) by a dentist (101, 102);
• communicating with said at least one dental specialist (201, 202) by the dentist (101, 102) over a communications network (40);
• generating a planning for the dental treatment;
• providing patient-specific information for simulating the dental treatment;
• creating a simulation of the dental treatment using a system incorporating online implant simulation software and online communication tools and being based on said patient-specific information;
• providing instructions to said dentist, said instructions being the dental treatment simulation provided by said at least one dental specialist; and
• accepting the simulation by the dentist (101, 102), wherein based on the accepted simulation, the system inventories the components and tools required to treat the patient.
**characterized in that**:
the simulation is being performed by interactively simulating treatment in function of known indications or contraindications comprising a determination of the most optimal positions for implants and abutments, in function of biomedical and aesthetic considerations.

8. Method according to claim 7 wherein said dentist and said at least one dental specialist are located at different sites.

9. Method according to claim 7 or claim 8 further comprising the steps of:
• reviewing said simulation of said dental treatment;
• declining said simulation of said dental treatment;
• modifying said simulation of said dental treatment, before said accepting of the simulation by the dentist.

10. Method according to any one of claims 7to 9wherein said selecting at least one dental specialist by a dentist comprises assisting said dentist by proposing at least one preferred dental specialist.

11. Method according to any one of claims 7 to 10 wherein said generating a planning for the dental treatment is based on availability of said at least one dental specialist.

12. Method according to any one of claims 7 to 11 further comprising reserving capacity of said at least one dental specialist.

13. Method according to any one of claims 7 to 12 wherein said providing patient-specific information comprises controlling access to said patient-specific information.

14. A computer program comprising instructions for carrying out the steps of the method according to any one of claims 7 to 13, when said computer program is executed.

15. A machine readable signal recording device on which is stored the computer program of claim 14.

## Patentansprüche

1. Computerbasiertes System (100) zur interaktiven Simulation einer Zahnbehandlung, die an einem Patienten vorzunehmen ist, wobei das Computersystem (100) mit einem Kommunikationsnetzwerk (40) verbunden ist und eine Datenspeichereinheit (32) zum Speichern von patientenspezifischen Informationen umfasst, wobei das Computersystem (100) umfasst:
• Mittel zum Auswählen mindestens eines Zahnspezialisten (201, 202) durch einen Zahnarzt (101, 102);
• Mittel zum Kommunizieren mit dem mindestens einen Zahnspezialisten (201, 202) durch den Zahnarzt (101, 102) über das Kommunikationsnetzwerk (40);
• Mittel zum Erzeugen einer Planung für die Zahnbehandlung;
• Mittel zum Abrufen der patientenspezifischen Informationen von der Datenspeichereinheit (32);
• Mittel zum Erzeugen einer Simulation der Zahnbehandlung auf der Basis der patientenspezifischen Informationen;
• Mittel zum Bereitstellen von Anweisungen an den Zahnarzt, wobei es sich bei den Anweisungen um die Zahnbehandlungssimulation handelt, die von dem mindestens einen Zahnspezialisten bereitgestellt wird;
• Mittel zum Akzeptieren der Simulation durch den Zahnarzt (101, 102), und
• Mittel zum Erzeugen einer Aufstellung von Vorrichtungen, die für die Zahnbehandlung benötigt werden, auf der Grundlage der akzeptierten Simulation,
**dadurch gekennzeichnet, dass**:
das System Online-Implantationssimulations-Software und Online-Kommunikationswerkzeuge beinhaltet; und
die Simulation durchgeführt wird durch interaktives Simulieren einer Behandlung in Abhängigkeit von bekannten Indikationen oder Kontraindikationen, umfassend eine Bestimmung der optimalen Positionen für Implantate und Abutments in Abhängigkeit von biomedizinischen und ästhetischen Erwägungen.

2. Computerbasiertes System nach Anspruch 1, wobei sich der Zahnarzt und der mindestens eine Zahnspezialist an unterschiedlichen Orten befinden.

3. Computerbasiertes System nach einem der vorhergehenden Ansprüche, ferner umfassend:
• Mittel zum Überprüfen der Simulation der Zahnbehandlung;
• Mittel zum Ablehnen der Simulation der Zahnbehandlung;
• Mittel zum Modifizieren der abgelehnten Simulation der Zahnbehandlung, oder
• Zugangskontrolle zu den patientenspezifischen Informationen, oder
• bei der Aufstellung der Vorrichtungen handelt es sich um Operations-Kits und personalisierte Anleitungen, die für die Zahnbehandlung benötigt werden.

4. Computerbasiertes System nach einem der vorhergehenden Ansprüche, ferner umfassend Mittel, um dem Zahnarzt mindestens einen bevorzugten Zahnspezialisten vorzuschlagen.

5. Computerbasiertes System nach einem der vorhergehenden Ansprüche, ferner umfassend eine Zugangskontrolle zu den patientenspezifischen Informationen.

6. Computerbasiertes System nach einem der vorhergehenden Ansprüche, wobei das Kommunikationsnetzwerk (40) ein Fernbereichsnetz ("Wide Area Network") ist.

7. Computerbasiertes Verfahren zur interaktiven Simulation einer Zahnbehandlung, die an einem Patienten vorzunehmen ist, wobei das Verfahren die Schritte umfasst:
• Auswählen mindestens eines Zahnspezialisten (201, 202) durch einen Zahnarzt (101, 102);
• Kommunizieren mit dem mindestens einen Zahnspezialisten (201, 202) durch den Zahnarzt (101, 102) über ein Kommunikationsnetzwerk (40);
• Erzeugen einer Planung für die Zahnbehandlung;
• Bereitstellen von patientenspezifischen Informationen zum Simulieren der Zahnbehandlung;
• Erzeugen einer Simulation der Zahnbehandlung unter Verwendung eines Systems, welches Online-Implantationssimulations-Software und Online-Kommunikationswerkzeuge beinhaltet, und auf der Basis der patientenspezifischen Informationen;
• Bereitstellen von Anweisungen an den Zahnarzt, wobei es sich bei den Anweisungen um die Zahnbehandlungssimulation handelt, die von dem mindestens einen Zahnspezialisten bereitgestellt wird; und
• Akzeptieren der Simulation durch den Zahnarzt (101,102), wobei das System auf der Grundlage der akzeptierten Simulation eine Aufstellung der Komponenten und Werkzeuge erstellt, die für die Zahnbehandlung benötigt werden,
**dadurch gekennzeichnet, dass**
die Simulation durchgeführt wird durch interaktives Simulieren einer Behandlung in Abhängigkeit von bekannten Indikationen oder Kontraindikationen, umfassend eine Bestimmung der optimalen Positionen für Implantate und Abutments in Abhängigkeit von biomedizinischen und ästhetischen Erwägungen.

8. Verfahren nach Anspruch 7, wobei sich der Zahnarzt und der mindestens eine Zahnspezialist an unterschiedlichen Orten befinden.

9. Verfahren nach Anspruch 7 oder Anspruch 8, ferner umfassend die Schritte:
• Überprüfen der Simulation der Zahnbehandlung;
• Ablehnen der Simulation der Zahnbehandlung;
• Modifizieren der Simulation der Zahnbehandlung vor dem Akzeptieren der Simulation durch den Zahnarzt.

10. Verfahren nach einem der Ansprüche 7 bis 9, wobei das Auswählen des mindestens einen Zahnspezialisten durch einen Zahnarzt Unterstützen des Zahnarztes durch Vorschlagen mindestens eines bevorzugten Zahnspezialisten umfasst.

11. Verfahren nach einem der Ansprüche 7 bis 10, wobei das Erzeugen einer Planung für die Zahnbehandlung auf der Verfügbarkeit des mindestens einen Zahnspezialisten basiert.

12. Verfahren nach einem der Ansprüche 7 bis 11, ferner umfassend Reservieren von Kapazität des mindestens einen Zahnspezialisten umfasst.

13. Verfahren nach einem der Ansprüche 7 bis 12, wobei das Bereitstellen von patientenspezifischen Informationen Kontrollieren des Zugangs zu den patientenspezifischen Informationen umfasst.

14. Computerprogramm, umfassend Befehle zum Durchführen der Schritte des Verfahrens nach einem der Ansprüche 7 bis 13, wenn das Computerprogramm ausgeführt wird.

15. Maschinenlesbare Signalaufzeichnungsvorrichtung, auf welcher das Computerprogramm nach Anspruch 14 gespeichert ist.

## Revendications

1. Système à base d'ordinateur (100) pour de la simulation interactive d'un traitement dentaire devant être administré à un patient, le système informatique (100) étant couplé à un réseau de communication (40) et comprenant une unité de stockage de données (32) pour stocker des informations spécifiques au patient, le système informatique (100) comprenant :
• un moyen pour sélectionner au moins un spécialiste dentaire (201, 202) par un dentiste (101, 102) ;
• un moyen pour communiquer avec ledit au moins un spécialiste dentaire (201, 202) par le dentiste (101, 102) sur ledit réseau de communication (40) ;
• un moyen pour générer une planification pour le traitement dentaire ;
• un moyen pour retrouver lesdites informations spécifiques aux patients à partir de ladite unité de stockage de données (32) ;
• un moyen pour créer une simulation du traitement dentaire sur la base desdites informations spécifiques au patient;
• un moyen pour fournir des instructions audit dentiste, lesdites instructions étant la simulation de traitement dentaire fournie par ledit au moins un spécialiste dentaire ;
• un moyen pour accepter la simulation par le dentiste (101, 102), et
• un moyen pour générer, sur la base de ladite simulation acceptée, un inventaire de dispositifs requis pour ledit traitement dentaire, **caractérisé en ce que** :
le système incorpore un logiciel de simulation d'implant en ligne et des outils de communication en ligne ; et
la simulation étant effectuée en simulant de façon interactive un traitement en fonction d'indications ou de contre-indications connues comprenant une détermination des positions les plus optimales pour des implants et des piliers, en fonction de considérations biomédicales et esthétiques.

2. Système à base d'ordinateur selon la revendication 1, dans lequel ledit dentiste et ledit au moins un spécialiste dentaire sont situés en des sites différents.

3. Système à base d'ordinateur selon l'une quelconque revendication précédente, comprenant en outre :
• un moyen pour examiner ladite simulation dudit traitement dentaire ;
• un moyen pour refuser ladite simulation dudit traitement dentaire ;
• un moyen pour modifier ladite simulation refusée dudit traitement dentaire, ou
• une commande d'accès auxdites informations spécifiques au patient, ou
• l'inventaire de dispositifs étant des kits chirurgicaux et des guides personnalisés, requis pour ledit traitement dentaire.

4. Système à base d'ordinateur, selon l'une quelconque revendication précédente, comprenant en outre un moyen pour proposer au moins un spécialiste dentaire préféré audit dentiste.

5. Système à base d'ordinateur selon l'une quelconque revendication précédente, comprenant en outre une commande d'accès auxdites informations spécifiques au patient.

6. Système à base d'ordinateur selon l'une quelconque revendication précédente, dans lequel ledit réseau de communication (40) est un réseau étendu.

7. Procédé à base d'ordinateur pour de la simulation interactive d'un traitement dentaire devant être administré à un patient, le procédé comprenant les étapes consistant à :
• sélectionner au moins un spécialiste dentaire (201, 202) par un dentiste (101, 102) ;
• communiquer avec ledit au moins un spécialiste dentaire (201, 202) par le dentiste (101, 102) sur un réseau de communication (40) ;
• générer une planification pour le traitement dentaire ;
• fournir des informations spécifiques au patient pour simuler le traitement dentaire ;
• créer une simulation du traitement dentaire en utilisant un système incorporant un logiciel de simulation d'implant en ligne et des outils de communication en ligne et étant basée sur lesdites informations spécifiques au patient ;
• fournir des instructions audit dentiste, lesdites instructions étant la simulation de traitement dentaire fournie par ledit au moins un spécialiste dentaire ; et
• accepter la simulation par le dentiste (101, 102), dans laquelle en se basant sur la simulation acceptée, le système inventorie les composants et outils requis pour traiter le patient ;
**caractérisé en ce que** :
la simulation est effectuée en simulant de façon interactive un traitement en fonction d'indications ou de contre-indications connues comprenant une détermination des positions les plus optimales pour des implants et des piliers, en fonction de considérations biomédicales et esthétiques.

8. Procédé selon la revendication 7, dans lequel ledit dentiste et ledit au moins un spécialiste dentaire sont situés en des sites différents.

9. Procédé selon la revendication 7 ou la revendication 8, comprenant en outre les étapes consistant à :
• examiner ladite simulation dudit traitement dentaire ;
• refuser ladite simulation dudit traitement dentaire ;
• modifier ladite simulation dudit traitement dentaire, avant ladite acceptation de la simulation par le dentiste.

10. Procédé selon l'une quelconque des revendications 7 à 9, dans lequel ladite étape consistant à sélectionner au moins un spécialiste dentaire par un dentiste comprend l'aide audit dentiste en proposant au moins un spécialiste dentaire préféré.

11. Procédé selon l'une quelconque des revendications 7 à 10, dans lequel ladite étape consistant à générer une planification pour le traitement dentaire est basée sur la disponibilité dudit spécialiste dentaire.

12. Procédé selon l'une quelconque des revendications 7 à 11, comprenant en outre l'étape consistant à réserver une capacité dudit spécialiste dentaire.

13. Procédé selon l'une quelconque des revendications 7 à 12, dans lequel ladite étape consistant à fournir des informations spécifiques au patient comprend la commande d'accès auxdites informations spécifiques au patient.

14. Programme informatique comprenant des instructions pour effectuer les étapes du procédé selon l'une quelconque des revendications 7 à 13, lorsque ledit programme informatique est exécuté.

15. Dispositif d'enregistrement de signal lisible par une machine sur lequel est stocké le programme informatique de la revendication 14.
